# EUROPEAN PATENT APPLICATION

(11) **EP 0 715 865 A2**
(43) Date of publication of application: **12.06.1996**
(21) Application number: 95308926.5
(22) Date of filing: 08.12.1995
(51) Int. Cl.: A61N 1/05

(54) **Steerable stylet assembly**

(30) Priority: 09.12.1994 US 353555
(71) Applicant: TELECTRONICS N.V., Willemstad Curaçao (AN)
(72) Inventor: Doumenis, Demetrious, Miami, Florida 33144 (US); Rebell, Allan K., North Miami, Florida 33168 (US)
(74) Representative: Waldren, Robin Michael

(57) **Abstract**

A steerable stylet assembly for use in guiding a medical catheter through a patient's venous system is provided. The device generally includes a handle assembly having an elongated inner stylet fixedly coupled thereto and an elongated outer stylet slideably coupled thereto. The elongated inner stylet includes a predefined non-linear shape and the elongated outer tube element includes a predefined linear shape. Accordingly, when the outer tube element envelopes the inner stylet, the device is linear; however, when the outer stylet is slid relative to the inner stylet it allows the inner stylet to take the predefined shape. Thus, the medical catheter takes the predefined shape. An optional mechanism is provided for use with active fixation medical catheters.

## Description

### Background of the Invention

The present invention is directed to steerable stylets, and, in particular, to a steerable stylet assembly for use during the implantation of a tissue-stimulating lead in a patient's body.

A tissue-stimulating lead is used with a pulse generator, such as an implantable cardiac pacemaker, in order to electrically couple the pulse generator to a desired tissue location in a patient's body. For example, when the pulse generator is a cardiac pacemaker, the tissue-stimulating lead, also referred to as a "pacing lead," electrically connects the pacemaker output circuit with an electrode that is positioned adjacent to the heart tissue to be stimulated. Access to the heart tissue is usually achieved transvenously. This is accomplished by inserting the lead into the heart through a major vein, such as the superior vena cava, thereby avoiding the trauma of open heart surgery.

A tissue-stimulating lead generally includes a connector at the proximal end for connecting the lead to the stimulating device (the pulse generator). At the distal end, an electrode is provided for delivering electrical pulses to body tissue. A flexible insulated conductor connects the proximal end with the distal end in order to transmit the electrical stimulating pulse from the tissue-stimulating device to the electrode. A lumen is often provided within the main body of the lead to receive a stylet for guiding the lead through the patient's venous system. The stylet is required for introducing an element of rigidity during implantation because the lead itself is designed to be highly flexible, so as not to disturb motion within the body after the initial implantation.

After a lead is guided through the venous system into the patient's heart, an electrophysiologist may use the lead to map the heart. This process involves moving the distal electrode tip to a plurality of different positions within the heart and measuring the different electrical signals at different points on the patient's heart tissue. This process is facilitated by providing a stylet that allows selective movement of the lead within the heart. The resulting information is sometimes used to determine the best location for an electrode tip to be secured to the cardiac tissue.

Electrode fixation is accomplished in either a positive or passive manner. Positive fixation (also known as "active fixation" ) leads are provided with some type of mechanism for actively securing and holding the distal electrode tip of the lead against the body tissue. The most common type of active fixation lead includes a helical screw located at the distal end that is screwed into the body tissue. In contrast, passive fixation leads include tines near the distal electrode that are positioned to become intertwined with the trabeculae located inside the heart. Over time, the trabeculae completely surround the tines and secure the electrode to the heart tissue.

The lead types discussed above are well-known in the prior art. Active fixation leads are advantageous due to the more reliable tissue contact that is established and verified during the implantation process. Alternatively, passive fixation leads are advantageous due to their general ease of implantation. U.S. Patent No. 5,005,587 issued to Scott, and assigned to the assignee of the present invention, discloses braided electrode leads and catheters and methods for using the same. In particular, this patent discloses a generally flexible lead assembly that includes an active fixation screw at the distal end. An electrode is provided proximate the tip in the usual manner, and a large braided section is provided for defibrillation. The braided section is more rigid than a normal lead body, due to its mesh construction which is useful for defibrillation, but it does not provide sufficient rigidity throughout the length of the lead to help in the implantation process.

U.S. Patent No. 5,228,455 issued to Barcel discloses an implant tool for extendable/retractable positive fixation leads. This tool in essence is a stylet that is used with a positive fixation lead that includes a helical fixation screw at the distal end. The helical fixation screw is extended and retracted with the aid of a spring-type construction; a rigid stylet is inserted through the lead and bears against a mechanism that causes the fixation coil to be extended. The Barcel tool is essentially a stylet that is used for extending the fixation coil and locking it in place. This tool does not help in directing the electrode head of the lead to a desired fixation position, or to steering the lead through the venous system of the patient.

Heretofore in the prior art, when a lead is implanted in a patient's body, the lead is guided through the venous system by a conventional stylet. A conventional stylet normally comprises a length of relatively stiff but malleable wire which can be inserted into the lumen of the lead. In this way, the stylet provides the essential rigidity or backbone to the lead so that it can be pushed through the patient's veins. When the lead is required to curve, the stylet must be removed from the lead, and the stylet wire is then manually curved by the physician (unless a pre-curved stylet wire is available), with the stylet then being reinserted. If the curve in the wire is not of the proper shape, then the physician must remove the stylet from the lumen of the lead and rebend the wire to the appropriate shape. This process may have to be repeated multiple times before the physician achieves the desired curved shape. Since the lead is implanted within a patient, maintaining a sterile environment is extremely important, and each time a tool (stylet) is removed from the patient, changed in shape, and reinserted, it creates the possibility of contamination, not to mention that it increases the duration of the implantation procedure.

Accordingly, it is desirable to develop a steerable stylet for use in guiding a pacing lead through a patient's venous system, and for locating the electrode tip at a desired location, with a minimal amount of removal and reinsertion of the stylet.

### Summary Of The Invention

Generally speaking, in accordance with the instant invention, a steerable stylet for use in implanting a medical catheter is provided. The steerable stylet assembly takes many different forms according to the type of medical catheter that is being implanted. The most generic form of the steerable stylet assembly includes a longitudinally extending inner stylet. A longitudinally extending outer stylet selectively envelops the inner stylet and a mechanism is provided for selectively engaging the inner stylet and the outer tubular stylet and moving one of them in relation to the other. One of the inner stylet and the outer tubular stylet also includes a predefined shape, such that the relative motion of one of the stylets with regard to the other causes the lead in which the stylet assembly is inserted to substantially assume the predefined shape.

When an active fixation type medical catheter (lead) is implanted in a patient, the steerable stylet assembly may further include a second longitudinally extending inner stylet with a member on its distal end for engaging the fixation member of the active fixation lead and causing the helical fixation screw to engage the heart tissue.

In yet another embodiment of the present invention that is used for implanting active fixation leads, a member is provided for engaging the conductor to cause it to twist and extend the helical fixation coil so that it fixes to the patient's heart tissue.

In still another embodiment of the invention for implanting an active fixation catheter that includes a pre-J-shape, the stylet includes a straight tubular sheath which is rigid in order to provide stiffness to the catheter during implantation. The sheath is also stiff enough to straighten out the J-shaped catheter. Once in the heart, the tubular sheath is retracted, and the catheter takes on the J-shape. A flexible inner stylet having a screwdriver head is then extended to affix the helical screw of the catheter.

Accordingly, it is an object of the invention to provide an improved stylet assembly for guiding a pacing lead through a patient's venous system and guiding the distal lead tip to the desired location in the patient's heart.

A further object of the invention is to provide a device that simplifies the lead implanting procedure.

Yet another object of the invention is to provide a steerable stylet device that allows the implanting physician to direct the distal end of the lead in any predesired direction.

Yet another object of the invention is to provide a stylet that increases the ability of the surgeon to map the heart with the lead prior to fixation.

Still another object of the invention is to provide an improved stylet that reduces implant time by controlling the shape and direction of the distal end of the lead assembly.

Still a further object of the invention is to allow for a more flexible lead either with or without a permanent J-shape and still allow for rotation of the helical fixation screw.

Still another object of the invention is to provide a more sterile lead by reducing the frequency of passing the stylet through the lumen.

Still other objects and advantages of the invention will in part be obvious and will in part be apparent from the specification.

The invention accordingly comprises the features of construction, combination of elements and arrangement of parts which will be exemplified in the constructions hereinafter set forth, and the scope of the invention will be indicated in the claims.

### Brief Description Of The Drawings

For a fuller understanding of the invention, reference is made to the following description, taken in connection with the accompanying drawings (which are not all to the same scale), in which:
FIG. 1 is a diagrammatic view of the steerable stylet in use during implantation of a lead into a patient's heart;
FIG. 2 is an elevational view of the proximal end of a preferred embodiment of the steerable stylet assembly, showing the proximal end of an active fixation lead coupled thereto;
FIG. 3 is a partial elevational view of a distal end of an active fixation lead;
FIG. 4 is a sectional view taken along line 4-4 of FIG. 2;
FIG. 5 is a sectional view taken along line 5-5 of FIG. 3;
FIG. 6 is a sectional view taken along line 6-6 of FIG. 5;
FIG. 7 is an elevational view of a steerable stylet assembly with the straight tubular sheath covering the J-shaped stylet wire which is inserted within the lumen of a medical catheter (lead), such that the lead takes the straight shape of the tubular sheath;
FIG. 8 is an elevational view of a steerable stylet assembly with the straight sheath pulled in the direction of arrow A and exposing a J-shaped stylet wire which is inserted within the lumen of a medical catheter (lead), such that the lead takes on the J-shape of the stylet wire;
FIG. 9 is an enlarged partial sectional view of the connection between the steerable stylet and the lead with the lead cut away to expose the J-shaped stylet wire;
FIG. 10 is a fragmentary view of an alternative embodiment of the steerable stylet for use with an active fixation lead;
FIG. 11 is a fragmentary view of the distal end of the stylet in accordance with the first embodiment of the invention; and
FIG. 12 is a fragmentary view of the distal end of the stylet in accordance with a further alternative embodiment of the invention.

### Detailed Description Of The Preferred Embodiments

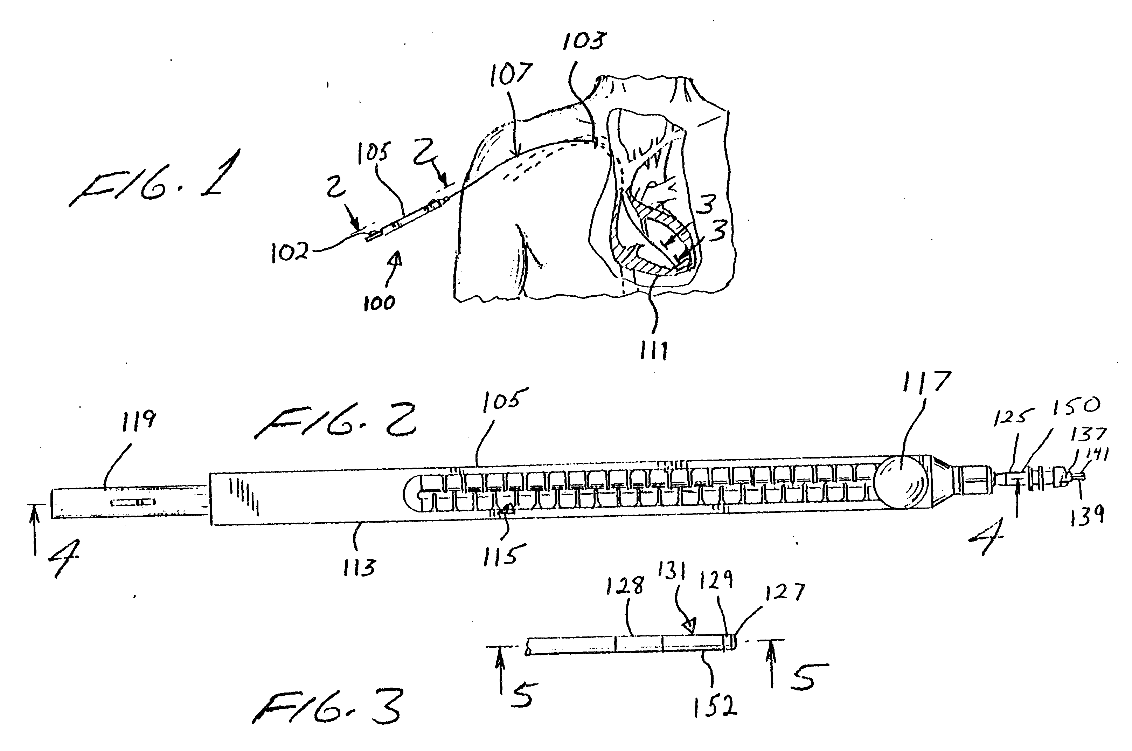

Reference is now made to FIG. 1 wherein a steerable stylet assembly, generally indicated as 100, constructed in accordance with the instant invention, is depicted. Steerable stylet 100 generally includes a proximal end 102 and a distal end 103. A handle 105 is located on proximal end 102 and a guide wire, generally indicated at 107, is located distal therefrom and terminates at distal end 103.

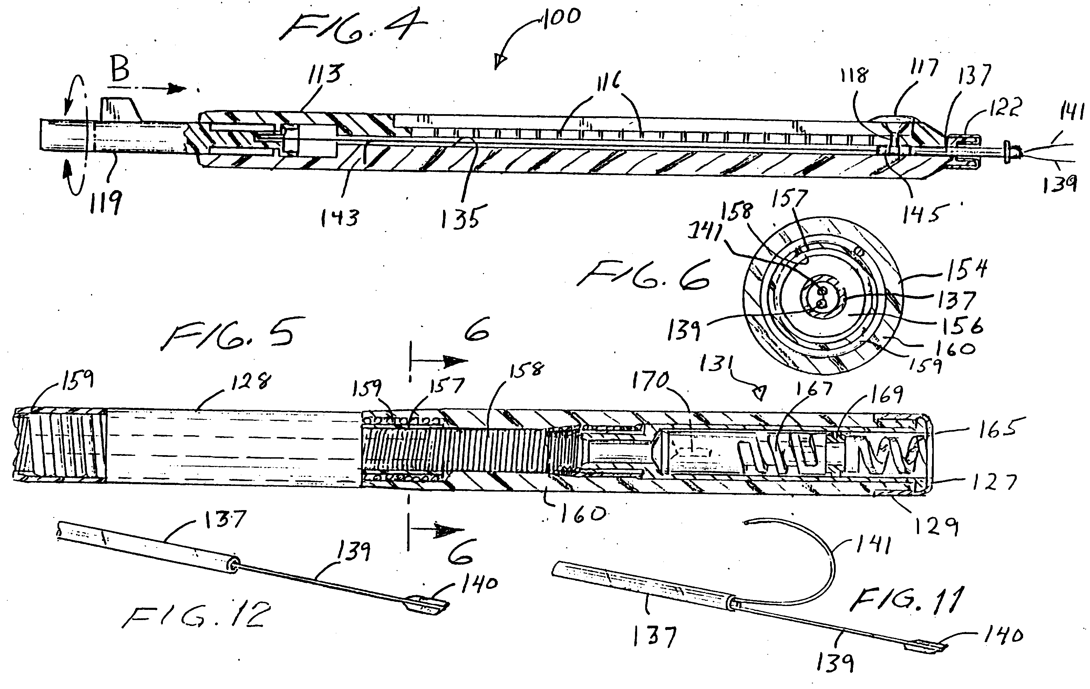

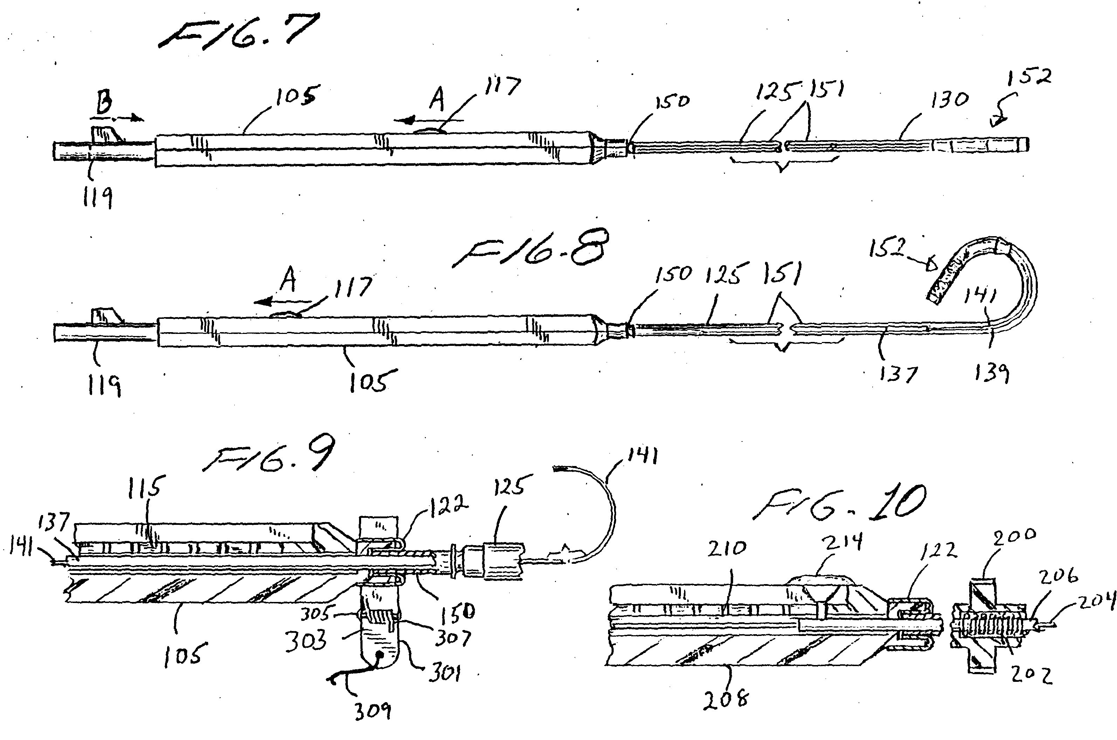

Although not shown in FIG. 1, a stimulating lead is located about the stylet guide wire 107, as shown more clearly in FIGS. 2 and 3. FIGS. 2 and 3 depict the stylet assembly in elevational view with a tissue-stimulating lead disposed about the stylet guide wire 107. Handle 105 generally includes a housing 113 having a ratchet section, generally indicated at 115, and a control knob 117 that works in conjunction with ratchet section 115. At the proximal end of stylet 100 a screwdriver control knob 119 is provided for controlling the operation of the screwdriver head discussed below. At the distal end of the housing 113, a tissue-stimulating lead 125 bears against housing 113 at terminal clip 122, and the stylet guide wire 107 is fed through the lead lumen (a hollow chamber in the center of the lead).

With particular reference to FIG. 3, the distal end of the lead is depicted. The pacing electrode, in the form of a tip 127, is provided with a conventional steroid eluting ceramic ring 129. This electrode lead is generally known as an active fixation lead and is generally indicated at 131, and is discussed in more detail in connection with FIG. 6. A second ring electrode 128 serves as a sensor in this bipolar lead configuration.

Particular reference is now made to FIGS. 4-8, wherein the stylet assembly 100 is shown in more detail. Stylet assembly 100 includes a housing 113 that defines a channel 135. A sheath 137 is disposed partially within channel 135. A first screwdriver stylet 139 is coupled at its proximal end to screwdriver control knob 119 and includes a screwdriver head 140 at its distal end. As shown in FIG. 4, the proximal end of screwdriver stylet 139 is embedded within the molded plastic screwdriver control knob 119, so that rotation of control knob 119 causes screwdriver head 140 to rotate. A rigid J-shaped stylet 141 is anchored to housing 113 by, for example, the proximal end 143 thereof being bent and embedded within housing 113 as depicted in FIG. 4. J-shaped stylet 141 does not move with respect to housing 113.

Sheath 137 is a somewhat rigid tubular member that envelops screwdriver stylet 139 and J-shaped stylet 141. Sheath 137 is coupled to control knob 117 by stem 118 which includes an annular coupling 145 for connecting the stem 118 to sheath 137. In the preferred embodiment, the stem includes a bore sized to receive sheath 137 therein, and two annular locking members are provided for locking and attaching annular coupling 145 in place. Thus, when control knob 117 is moved in an axial direction from the distal end of housing 113 to the proximal end of housing 113, or vice versa, the sheath moves along with control knob 117. A plurality of teeth 116 engage shaft 118 of knob 117 when the knob is moved along the axial direction. These teeth provide the function of locking the control knob 117 in place. Thus, sheath 137 is also locked in place. As the sheath is moved in the proximal direction (see FIG. 11), the J-shaped wire 141 becomes exposed, and the tissue-stimulating lead 125 is bent in accordance with the J-shaped wire. As discussed hereinbelow, the sheath 137 is more rigid than the J-shaped wire; accordingly, the J-shaped wire is straightened when the sheath is pushed over the J-shaped wire.

With particular reference to FIGS. 2, 3, 5 and 6, the electrode lead 125 has a generally tubular elongated shape with a proximal portion 150, a mid-section or shaft portion 151 (shown cut away in FIGS. 7 and 8) and a distal electrode portion 152.

With particular reference to FIG. 6, the mid-section of the lead spans the entire length of the lead assembly 125, and is comprised of a flexible cylinder 154 having an inner channel or lumen 156 defined by a flexible, coiled wire conductor 158 and a flexible cylindrical shaft outer insulator 160. In a bipolar lead, as shown in FIG. 6, a second coiled wire conductor 159 is provided. An insulator 157 is disposed between coiled wire conductors 158 and 159. The stylet sheath 137 is inserted through the shaft inner channel 156 to engage the distal electrode portion of the lead assembly and facilitate its implantation in the patient's heart. Thus, the stylet assembly is used to guide a distal electrode tip 127 into an appropriate position within the heart tissue.

Tip electrode 127 is a porous electrode; the porosity provides greater surface area for contacting muscle tissue. A drug eluting ceramic element 129 is provided proximate thereto to prevent excess tissue growth or scarring in the area surrounding tip electrode 127, thereby decreasing the pacing threshold of the electrode. The internal mechanisms of the active fixation portion of the electrode are shown in detail in FIG. 5. This shows a helical fixation coil 165 connected to a compound screw 167, with a gasket 169 provided therebetween. The screwdriver stylet is inserted into the recessed receiving head 170 (shown in phantom), such that the compound screw 167 can be rotated by the physician using the screwdriver control knob 119 of stylet 100. The operation of the active fixation lead is shown in more detail in U.S. Patent No. 5,300,108, which issued on April 5, 1994, and is incorporated by reference herein.

FIG. 7 specifically illustrates the stylet with control knob 117 located in the distal direction. Accordingly, the sheath 137 substantially envelopes J-shaped wire 141 and screwdriver wire 139. However, the screwdriver head 141 extends past the outer sheath when screwdriver control knob 119 is pushed in the direction of arrow B of FIG. 4, such that it engages the receiving portion 170 shown in FIG. 5. When sheath 137 is fully extended over J-shaped wire 141, J-shaped wire 141 is caused to be straight because the sheath is more rigid than the J-shaped wire. However, when sheath 137 is retracted by the physician moving control knob 117 to a more proximal position, in the direction of arrow A of FIG. 8, then J-shaped wire 141 is exposed and causes the tissue-stimulating lead 125 to bend in accordance with the configuration of the J-shaped wire 141 as illustrated in FIG. 8. This is due to the fact that J-shaped wire 141 is more rigid than the tissue-stimulating lead 125. Accordingly, the physician can cause the lead to bend as little or as much as desired by moving the control knob 117 in the direction of arrow A (to be more bent), or opposite to the direction of arrow A (to be straightened out).

When a physician is implanting a lead within a patient's body, the lead is designed to be as flexible as possible, since it will be retained within the venous system of the patient. Thus, a stylet of the type disclosed herein assists the physician in implanting the lead, by providing the required rigidity to the lead when being implanted. When the physician comes to a bend in the vein, the stylet is selectively bent by moving the control knob 117. Furthermore, once lead 125 is completely within the patient's heart, the physician can selectively maneuver the distal electrode tip 127 to assure proper placement within the patient's heart. For example, the physician must extend the J-shape configuration substantially to the maximum level in order to contact the atrial appendage. Alternatively, only a slight bend is required when implanting a lead in the ventricular apex.

Particular reference is now made to FIG. 9, where a fragmentary cross-sectional view of a steerable stylet assembly is shown for use with a passive fixation lead. This stylet assembly includes a J-shaped wire 141, but does not include a screwdriver head stylet as in the first embodiment. As described above, this stylet would be used with a passive fixation lead that would include tines located near the distal electrode that are positioned to become intertwined with the trabeculae located inside the heart.

FIG. 9 illustrates the handle 105 of a steerable stylet 100. A tissue-stimulating lead 125 is shown partially cut away. Sheath 137 is also cut away, so that J-shaped wire (stylet) 141 is shown exposed. In this two-piece configuration that includes one J-shaped wire and an outer tubular sheath 137, the steerable stylet functions identical to the first embodiment which includes two-wire stylets and a tubular sheath.

With reference to FIGS. 4, 9 and 10, the terminal clip 122 located at the distal end of housing 113 is formed of metal or any other conductor. The metallic terminal clip 122 is designed to be in contact with the metallic proximal end 150 of tissue stimulating lead assembly 125. The metallic proximal end 150 of lead assembly 125 is the portion of the lead that is plugged into the pulse generator to form a pacing device.

In FIG. 9, a clip 301 is a conventional alligator clip which includes a body portion 303, a pivot pin 305 and a helical biasing spring 307. Clip 301 is formed of a metal or other conductor and has a conductive wire 309 electrically coupled thereto. In practice, conductive wire 309 is hooked up to an electronic meter (not shown) for determining the contact impedance between the electrode 127 and the tissue to be stimulated. This is commonly known as mapping the heart to deter-mine the best location for implanting the electrode. A clip such as clip 301 can be electrically coupled to the terminal clip 122 in any of the embodiments of this invention, and is shown connected to terminal clip 122 of FIG. 9 for illustrative purposes.

Particular reference is next made to FIG. 10 which illustrates an alternative embodiment of the invention for use with an active fixation lead that does not include a screwdriver driven mechanism. This active fixation lead requires the stylet assembly to include a mechanism 200 to rotate the coiled wire 202. The coiled wire 202 is coupled to the active fixation device (not shown) in a conventional manner. In this embodiment, a steerable stylet for an active fixation lead includes a J-shaped wire 204 and a sheath 206. The stylet body 208 includes a rachet section 210 having teeth 212 to interact with control knob 214 which functions as described above with regard to the first embodiment. The control knob 214 is coupled to sheath 206 as described above in connection with the first embodiment of the invention. Thus, this embodiment differs from the first embodiment in that mechanism 200 is required for engaging coiled wire 202 which then activates the active fixation lead.

FIG. 11 is an enlarged fragmentary view of the distal end of the three-piece steerable stylet with the sheath 137 displaced in the direction of Arrow A of FIG. 8, so that the J-shaped wire 141 and screwdriver wire 139 are exposed. During operation, when the control knob 117 is moved in the direction of Arrow A of FIG. 8, the sheath moves linearly therewith and exposes the J-shaped wire so that the tissue-stimulating lead 125 assumes the J-shape of J-shaped wire stylet 141. The screwdriver stylet 139 is less rigid than J-shaped wire stylet 141 and conforms to the shape of J-shaped wire stylet 141.

An alternative inverted embodiment is possible, where the sheath includes a J-shape configuration, and the internal rigid wire stylet is straight. In this embodiment, the control knob would be connected to the rigid wire stylet, and the tubular sheath would be fixed in place relative to the stylet handle. Thus, when the wire extends to the end of the sheath the stylet and thus the tissue stimulating lead would extend in a straight direction. Alternatively, when the control knob is moved and the rigid wire stylet is guided away from the distal end, then the distal end of the sheath would take on a predefined J-shaped configuration. Thus, the function of the device is similar to that of the first embodiment; however, it is inverted. Once again, a flexible screwdriver stylet can be included for a three-piece configuration.

Accordingly, the present invention contemplates a new stylet assembly that generally includes at least a two-piece configuration using an inner shaped stylet and an outer tubular sheath. In the first preferred embodiment, the sheath is more rigid than the inner wire stylet. A handle assembly is provided with a member for moving the sheath relative to the wire stylet so that the J-shaped wire is selectively exposed to engage and shape a tissue-stimulating lead that surrounds the sheath. This device eliminates the need to insert and remove a stylet from the lumen of a tissue stimulating lead during implantation.

Furthermore, an optional flexible screwdriver stylet may be incorporated within the lead for use during implantation. The screwdriver knob is incorporated on the handle of the stylet to provide means for an implanting physician to affix the fixation coil to the patient.

During implantation, a physician inserts the steerable stylet within the lumen of an appropriate pacing lead. The stylet provides the lead with the required stiffness to be implanted through the venous system. The stylet can be selectively bent and twisted to facilitate implantation. Once the distal pacing electrode is in the patient's heart, the physician can then place the electrode at the desired position by moving control knob 117 and twisting the handle assembly. If an active fixation lead is implanted, an appropriate tool for activating the lead is incorporated in the stylet assembly; for example, the screwdriver stylet of the first embodiment.

Particular reference is next made to FIG. 12 which illustrates another alternative embodiment of the invention. The embodiment of FIG. 12 includes the same stylet housing configuration as the embodiments of FIGS. 1-10. However, the embodiment of FIG. 12 is for use with a pre-J-shaped active fixation lead. Thus, the J-shaped stylet wire is not required. The tubular sheath 137 is rigid and straight. Thus, when sheath 137 is inserted within a pre-J-shaped lead the lead is straightened, and is provided with the rigidity required for implantation within the patient's body. Once the lead is within the patient's heart, the sheath 137 is retracted as shown in FIG. 12, and the J-shaped lead automatically takes on the J-shape. At this point, the soft screwdriver stylet 139 with screwdriver head 140 can operate of the active fixation lead as described above. Thus, when a J-shaped lead is used, no J-shaped stylet wire is required.

It will thus be seen that the objects set forth above, among those made apparent from the preceding description, are efficiently attained and, since certain changes may be made in carrying out the above method and in the constructions set forth without departing from the spirit and scope of the invention, it is intended that all matter contained in the above description and shown in the accompanying drawings shall be interpreted as illustrative and not in a limiting sense.

It is also to be understood that the following claims are intended to cover all of the generic and specific features of the invention herein described, and all statements of the scope of the invention which, as a matter of language might be said to fall therebetween.

## Claims

1. A steerable stylet assembly for use in guiding a medical catheter through a patient's venous system, comprising:
a first elongated stylet element;
an elongated outer tube element surrounding said stylet element and being slidable therein;
one of said elements having a predefined non-linear shape and the other of said elements having a linear shape; and
means for moving said stylet element and said tube element relative to each other such that said catheter assumes said predefined shape.

2. The steerable stylet assembly of claim 1 wherein said steerable stylet assembly includes a handle assembly.

3. The steerable stylet assembly of claim 2 wherein said first stylet element is fixedly secured to said handle assembly.

4. The steerable stylet assembly of claim 3 wherein said tube element is slidable relative to said handle assembly.

5. The steerable stylet assembly of claim 2 wherein said tube element is slidable relative to said handle assembly.

6. The steerable stylet assembly of claim 2 wherein said handle assembly includes means for controlling a said medical catheter having an active fixation lead.

7. The steerable stylet assembly of claim 6 wherein said medical catheter having an active fixation lead includes at least one coiled conductor extending through said catheter and operatively coupled to said active fixation lead, and said controlling means is a device for engaging the coiled wire of the medical catheter and facilitating the twisting of the coiled wire.

8. The steerable stylet assembly of claim 6 wherein said controlling means is a second elongated stylet having a proximal end and a distal end.

9. The steerable stylet assembly of claim 8 wherein said proximal end of said second elongated stylet is rotationally coupled to said housing, and said distal end of said second elongated stylet includes a screwdriver head.

10. The steerable stylet assembly of claim 1 wherein said moving means includes a control element coupled to said tube element.

11. The steerable stylet assembly of claim 10 wherein said steerable stylet assembly includes a handle assembly defining a track.

12. The steerable stylet assembly of claim 11 wherein said control element is disposed partial within said track and movement of said control element along said track causes said tube element to move relative to said first stylet element.

13. The steerable stylet assembly of claim 1 wherein said first elongated stylet element includes a predefined J-shape.

14. The steerable stylet assembly of claim 13 wherein said elongated outer tube element is linear.

15. The steerable stylet assembly of claim 14 wherein said elongated outer tube element is more rigid than said first elongated stylet element

16. The steerable stylet assembly of claim 14 further including a handle assembly fixedly coupled to said first elongated stylet.

17. The steerable stylet assembly of claim 16 wherein said elongated outer tube element is slidable relative to said housing and said first elongated stylet.

18. The steerable stylet assembly of claim 1 wherein said elongated outer tube element includes a predefined J-shape.

19. The steerable stylet assembly of claim 8 wherein said first elongated stylet element is linear.

20. The steerable stylet assembly of claim 19 wherein said first elongated stylet element is more rigid than said outer tube element.

21. The steerable stylet assembly of claim 19 further including a handle assembly fixedly coupled to said elongated outer tube element.

22. The steerable stylet assembly of claim 21 wherein said first elongated stylet element is slidable relative to said housing and said elongated outer tube element.

23. A steerable stylet assembly for use in implanting an active fixation lead into a patient, said active fixation lead including an elongated flexible lead body, having a proximal end and a distal end, said distal end including an active fixation device, and said lead body defining a lumen, comprising:
a first elongated inner stylet element having a proximal end and a distal end, and having a predefined non-linear shape along at least a portion of said distal end;
a second elongated inner stylet element having a proximal end and a distal end, and having means for selectively extending said active fixation device;
an elongated outer tube element surrounding said first and second inner stylet elements, and having a predefined linear shape; and
means for moving said outer tube element relative to said first elongated inner stylet element.

24. The steerable stylet assembly of claim 23 wherein said first elongated inner stylet is more rigid than said second elongated inner stylet.

25. The steerable stylet assembly of claim 24 wherein said elongated outer tube element is more rigid than said first inner stylet element, so that the elongated outer tube element maintains the predefined linear shape at all times.

26. The steerable stylet assembly of claim 23 wherein said steerable stylet assembly includes a handle assembly.

27. The steerable stylet assembly of claim 26 wherein said first elongated inner stylet element is fixedly secured to said handle assembly.

28. The steerable stylet assembly of claim 27 wherein said elongated outer tube element is slidable relative to said handle assembly.

29. The steerable stylet assembly of claim 28 wherein said proximal end of said second elongated stylet is rotationally coupled to said housing.

30. The steerable stylet assembly of claim 26 wherein said handle assembly defines a track.

31. The steerable stylet assembly of claim 30 wherein said moving means includes a control element coupled to said outer tube element.

32. The steerable stylet assembly of claim 31 wherein said control element is disposed partial within said track and movement of said control element along said track causes said tube element to move relative to said first elongated inner stylet element.

33. The steerable stylet assembly of claim 23 wherein said first elongated inner stylet element includes a predefined J-shape.

34. A system for implanting an active fixation lead into a patient, comprising an active fixation lead including an elongated flexible lead body having a proximal end and a distal end, said distal end including an active fixation device and having a predefined non-linear shape, and said lead body defining a lumen; a stylet assembly including an elongated inner stylet element having a proximal end and a distal end, said inner stylet element having means for selectively extending said active fixation device located on said distal end, an elongated outer tube element surrounding said inner stylet element, said outer tube element having a predefined essentially linear shape, and means for moving said outer tube element relative to said distal end of said active fixation lead.

35. The system of claim 34 wherein said outer tube stylet is more rigid than said predefined non-linear shaped lead, so that said lead is essentially linear when said outer tube stylet extends to the distal end of said lead, and is said predefined non-linear shape when said outer tube stylet is retracted from said distal end of said lead.

36. The system of claim 34 wherein said steerably stylet assembly includes a handle assembly.

37. The system of claim 36 where said tube element is slidable relative to said handle assembly.
